# EUROPEAN PATENT APPLICATION

(11) **EP 4 067 472 A1**
(43) Date of publication of application: **05.10.2022**
(21) Application number: 20906218.1
(22) Date of filing: 22.12.2020
(51) Int. Cl.: C12M 1/26, C12N 1/02, C12Q 1/04, G01N 33/48

(54) **EXTRACELLULAR VESICLE RECOVERY METHOD AND BLOOD COLLECTION TUBE**

(30) Priority: 23.12.2019 JP 2019231991
(71) Applicant: Fujirebio Inc., Shinjuku-ku Tokyo 163-0410 (JP); H.U. Group Research Institute G.K., Akiruno-shi, Tokyo 197-0833 (JP)
(72) Inventor: GU, Ran, Tokyo 163-0410 (JP); INUZUKA, Tatsutoshi, Tokyo 197-0833 (JP)
(74) Representative: Müller-Boré & Partner Patentanwälte PartG mbB
(86) International application number: PCT/JP2020/047855
(87) International publication number: WO 2021/132213

(57) **Abstract**

The present invention provides a method and means useful for recovering an extracellular vesicle. More specifically, the present invention provides the following (I) and (II).
(I) A method for recovering extracellular vesicle, the method including:
(1) mixing whole blood with a nonionic surfactant and a chelating agent to give a mixture solution containing the extracellular vesicle, the nonionic surfactant and the chelating agent; and
(2) separating the extracellular vesicle from the mixture solution.

(II) A blood collection vessel containing a nonionic surfactant and a chelating agent.

## Description

### FIELD

The present invention relates to a method for recovering an extracellular vesicle(s), and a blood collection vessel, and the like.

### BACKGROUND

An extracellular vesicle (EV) is a microscopic vesicle secreted from various types of cells and having a membrane structure, and exists in body fluids such as blood. Examples of the extracellular vesicles secreted extracellularly include exosomes, ectosomes, and apoptotic blebs. Since the extracellular vesicle contains various substances that play functions such as intercellular signaling, it is analyzed for the purposes of diagnosis, drug discovery and the like. Thus, it is required to develop a method and means useful for recovering the extracellular vesicles, which can be applied to such analyses.

For example, Patent Literature 1 discloses that a nonionic surfactant can reduce nonspecific adsorption in an immunoreaction between extracellular vesicles and an anti-CD antibody in a buffer (more specifically, an immunoreaction between an anti-CD antibody and an extracellular vesicle-containing buffer in which liquid mediums are exchanged from serum to a buffer by using magnetic particles and a washing buffer) (Test example 4 and Fig. 2).

Patent Literature 2 discloses that although recovery yield of the extracellular vesicle can be improved in the presence of EDTA at a low concentration less than approximately 3.0 mg/mL (approximately 8 mM), recovery yield of the extracellular vesicle cannot be improved or rather is significantly reduced in the presence of EDTA at a higher concentration (Example 1 and Fig.1A to 1C).

Meanwhile, plasma and serum can be prepared from whole blood, and are commonly used as blood samples in clinical blood testing. Hemolysis caused by disruption of red blood cells is widely known to affect results of blood tests, and supposedly needs to be avoided. In addition, blood collection vessels are widely used in clinical practice for processing whole blood to prepare blood samples from the viewpoint of convenience, promptness, and the like of processing.

### CITATION LIST

### PATENT LITERATURE

Patent Literature 1: WO2015/068772
Patent Literature 2: U.S. Patent Application Publication No. 2015/0125864A1 description

### SUMMARY OF INVENTION

### TECHNICAL PROBLEM

The present invention is aimed at providing a method and means useful for recovering an extracellular vesicle(s).

### SOLUTION TO PROBLEM

As a result of intensive investigation, the inventors of the present invention found that recovery yield of an extracellular vesicle(s) from whole blood can significantly be improved by mixing a nonionic surfactant and a chelating agent with whole blood.

It is generally recognized in clinical practice that use of surfactant in the processing of whole blood for blood testing should be avoided because surfactants disrupt lipid bilayers and thus cause hemolysis due to the disruption of red blood cells. However, since hemolysis can easily be avoided by selecting a nonionic surfactant (especially a specific nonionic surfactant) among surfactants to use, the use of such a nonionic surfactant in combination with a chelating agent can significantly improve the recovery yield of extracellular vesicles from whole blood and it is expected to reduce or avoid hemolysis. Therefore, the application of the nonionic surfactant and the chelating agent to whole blood enables preparation of a versatile blood sample that is used not only for the analysis of extracellular vesicles but also for ordinary blood tests.

As mentioned above, Patent Literature 1 discloses that the nonionic surfactant can reduce nonspecific adsorption in the immunoreaction between extracellular vesicles and anti-CD antibody in the buffer. However, Patent Literature 1 does not disclose that whole blood is mixed with the nonionic surfactant (the nonionic surfactant is allowed to act on the whole blood) and the recovery yield of the extracellular vesicle can be improved by the mixing. In addition, in light of the fact that the improvement in the recovery yield of the extracellular vesicles from whole blood by mixing the nonionic surfactant and the chelating agent with whole blood is considered to be another effect achieved independently from the reduction of nonspecific adsorption in the immune response (Example 5), Patent Literature 1 does not teach or suggest the present invention.

The inventors of the present invention also found that the use of both the nonionic surfactant and the chelating agent enables production of a blood collection vessel which can significantly improve the recovery yield of the extracellular vesicle from whole blood and a highly versatile blood collection vessel used not only for analysis of the extracellular vesicle but also for ordinary blood tests, and completed the present invention.

That is, the present invention is as follows.
[1] A method for recovering an extracellular vesicle(s), the method comprising:
   (1) mixing whole blood with a nonionic surfactant and a chelating agent to give a mixture solution containing the extracellular vesicle(s), the nonionic surfactant and the chelating agent; and
   (2) separating the extracellular vesicle(s) from the mixture solution.
[2] The method according to [1], wherein the nonionic surfactant is a nonionic surfactant having a polyoxyethylene alcohol structure.
[3] The method according to [2], wherein the nonionic surfactant having the polyoxyethylene alcohol structure is an alcohol ethoxylate.
[4] The method according to [2], wherein the nonionic surfactant having the polyoxyethylene alcohol structure is a polyoxyethylene-polyoxyalkylene block copolymer.
[5] The method according to any of [1] to [4], wherein the nonionic surfactant is a nonionic surfactant with an HLB of 18 or more.
[6] The method according to any of [1] to [5], wherein a concentration of the nonionic surfactant in the mixture solution is 0.01 to 10 weight/volume %.
[7] The method according to any of [1] to [6], wherein the chelating agent is hydroxyethyl iminodiacetic acid (HIDA), nitrilotriacetic acid (NTA), hydroxyethyl ethylenediaminetriacetic acid (HEDTA), ethylenediaminetetraacetic acid (EDTA), ethylenediaminetetra (methylene phosphonic acid) (EDTMP), glycol ether diamine tetraacetic acid (EGTA), or a salt thereof.
[8] The method according to any of [1] to [7], wherein a concentration of the chelating agent in the mixture solution is 10 mM to 1000 mM.
[9] The method according to any of [1] to [8], wherein the mixing is performed after the whole blood is placed in a blood collection vessel containing the nonionic surfactant and the chelating agent.
[10] The method according to any of [1] to [9], wherein the separating is performed by any of the following (A) to (C):
   (A) collection of a supernatant from the mixture solution;
   (B) use of a substance capable of binding to a surface marker of the extracellular vesicle(s) in the mixture solution; or
   (C) collection of the supernatant from the mixture solution and use of the substance capable of binding to the surface marker of the extracellular vesicle(s) in the supernatant.
[11] The method according to any of [1] to [10], wherein the extracellular vesicle is exosome.
[12] A method for analyzing an extracellular vesicle(s), the method comprising:
   (1) mixing whole blood with a nonionic surfactant and a chelating agent to give a mixture solution containing the extracellular vesicle(s), the nonionic surfactant and the chelating agent;
   (2) separating the extracellular vesicle(s) from the mixture solution; and
   (3) analyzing the extracellular vesicle(s).
[13] A blood collection vessel comprising a nonionic surfactant and a chelating agent.
[14] The blood collection vessel according to [13], wherein an amount of the nonionic surfactant in the blood collection vessel is 0.1 to 2000 mg.
[15] The blood collection vessel according to [13] or [14], wherein an amount of the chelating agent in the blood collection vessel is 3.8 to 3800 mg.

### EFFECTS OF INVENTION

The method of the present invention can significantly improve the recovery yield of the extracellular vesicle from whole blood, and is expected to reduce or avoid hemolysis.

The blood collection vessel of the present invention can significantly improve the recovery yield of the extracellular vesicle from whole blood, and is highly versatile in use not only for analysis of the extracellular vesicle but also for ordinary blood tests.

### EMBODIMENTS FOR CARRYING OUT INVENTION

The present invention provides a method for recovering an extracellular vesicle(s), including the following:
(1) mixing whole blood with a nonionic surfactant and a chelating agent to give a mixture solution containing the extracellular vesicle(s), the nonionic surfactant and the chelating agent; and
(2) separating the extracellular vesicle(s) from the mixture solution.

Whole blood can be collected from animals. Examples of animals include mammals (e.g., primates such as humans and monkeys; rodents such as mice, rats and rabbits; ungulates such as cattle, pigs, goats, horses and sheep, and carnivores such as dogs and cats) and birds (e.g., chickens). Preferably, the animal is a mammal such as a human.

Whole blood contains extracellular vesicles. Thus, whole blood is mixed with the nonionic surfactant and the chelating agent to give the mixture solution that contains the extracellular vesicles, the nonionic surfactant and the chelating agent.

The extracellular vesicle is a microscopic vesicle that is secreted from various types of cells and has a membrane structure. Examples of the extracellular vesicle include exosomes, ectosomes and apoptotic vesicles. Preferably, the extracellular vesicle is the exosome. The extracellular vesicle can also be defined by its size. The size of the extracellular vesicle is, for example, 30 to 1000 nm, preferably 50 to 300 nm, and more preferably 80 to 200 nm. The size of the extracellular vesicle can be measured by NanoSight (manufactured by Malvern Instruments).

In addition, the extracellular vesicle can be defined by an extracellular vesicle marker. Examples of the extracellular vesicle marker include an extracellular vesicle inside marker and an extracellular vesicle surface marker. Examples of the extracellular vesicle inside marker include carcinoembryonic antigen (CEA), CA125, CA15-3, actin family, TSG101, ALIX, Charged multivesicular body protein (CHMP) family, Glyceraldehyde 3-phosphate dehydrogenase (GAPDH), poly (ADP-ribose) polymerase (PARP) family, AFP, CA19-9, Flotillin-I, Flotillin-II, Rab protein, programmed cell death (PDCD) 6, phospholipid scramblase (PLSCR) family, cytochrome C oxidase (COX) family, lamin family, proliferating cell nuclear antigen (PCNA), tubulin family, TATA binding protein (TBP), voltage dependent anionic channel protein (VDAC), amyloid beta, and tau protein. Examples of the extracellular vesicle surface marker include a tetraspanin membrane protein (extracellular vesicle membrane specific four transmembrane proteins, e.g., CD9, CD63 and CD81), an extracellular matrix metalloproteinase inducer (e.g., CD147), heat shock protein (HSP) 70, HSP90, major histocompatibility complex (MHC) I, lysosome associated membrane protein (LAMP) 1, intercellular adhesion molecule (ICAM)-1, integrin, ceramide, cholesterol, phosphatidylserine, Annexins, Caveolin-I and EpCAM. The extracellular vesicle marker is preferably the extracellular vesicle surface marker, more preferably the tetraspanin membrane protein, and still more preferably CD9.

In the present invention, among the surfactants, the use of nonionic surfactant enables reduction or avoidance of hemolysis. Any nonionic surfactant can be used as the nonionic surfactant, and it is preferred to use a nonionic surfactant having a polyoxyethylene alcohol structure.

The nonionic surfactant having the polyoxyethylene alcohol structure is preferably a linear or branched chain compound containing a structure represented by -O-(-CH₂-CH₂-O-)ₓ-H (compound not containing cyclic structure). Here, x may be an integer of 1 or more, preferably an integer of 10 or more, more preferably an integer of 20 or more, still more preferably an integer of 30 or more, and particularly preferably an integer of 40 or more. As well, x may be an integer of 400 or less, preferably an integer of 350 or less, more preferably an integer of 300 or less, still more preferably an integer of 280 or less, and particularly preferably an integer of 270 or less. More specifically, x may be an integer of from 1 to 400, preferably an integer of from 10 to 350, more preferably an integer of from 20 to 300, still more preferably an integer of from 30 to 280, and particularly preferably an integer of from 40 to 270. Herein, the value of x represents an average value.

The nonionic surfactants having the polyoxyethylene alcohol structure may be formed by only single bonds or may contain an unsaturated bond(s) (double/triple bond(s)), and is preferably a compound formed by only single bonds. Such a compound may include alkyl structures (linear or branched chain), and/or polyoxyalkylene structures (linear or branched chain) as a structure(s) other than the polyoxyethylene alcohol structure. The alkyl structure may be a C₆₋₃₁ alkyl structure, for example, and is preferably a C₆₋₃₁ linear alkyl structure. The alkylene in the polyoxyalkylene structure may be a C₁₋₆ alkylene, for example. Examples of the C₁₋₆ alkylene include C₁ alkylene (methylene), C₂ alkylenes (ethylene group and ethylidene group), C₃ alkylenes (propylidene, propylene, trimethylene and isopropylidene), C₄ alkylenes (e.g., tetramethylene), C₅ alkylenes (e.g., pentamethylene), and C₆ alkylenes (e.g., hexamethylene). Such a compound may be composed of a carbon atom(s), a hydrogen atom(s), and an oxygen atom(s).

More specifically, examples of such a nonionic surfactant having the polyoxyethylene alcohol structure include alcohol ethoxylates and polyoxyethylene-polyoxyalkylene block copolymers (e.g., polyoxyethylene-polyoxypropylene block copolymers).

The alcohol ethoxylate is also referred to as a poly(oxyethylene) alkylether, and is a compound in which a hydrophilic polyoxyethylene (POE) chain(s) is linked to a hydrophobic alkyl group(s) via an ether bond(s). The alcohol ethoxylate can be represented by the following formula (1). 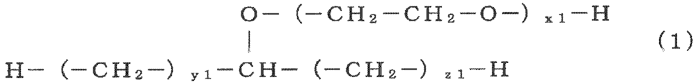
wherein x1 is an integer of 1 or more;
y1 is an integer of 0 or more;
z1 is an integer of 0 or more; and
y1 ≥ z1.

Herein, the values of x1, y1 and z1 represent average values.

x1 may be an integer of 1 or more, preferably an integer of 10 or more, more preferably an integer of 20 or more, still more preferably an integer of 30 or more, and particularly preferably an integer of 40 or more. As well, x1 may be an integer of 300 or less, preferably an integer of 250 or less, more preferably an integer of 200 or less, still more preferably an integer of 150 or less, and particularly preferably an integer of 100 or less, 80 or less, 60 or less, or 50 or less. More specifically, x1 may be an integer of from 1 to 300, preferably an integer of from 10 to 250, more preferably an integer of from 20 to 200, still more preferably an integer of from 30 to 150, and particularly preferably an integer of from 40 to 100, from 40 to 80, from 40 to 60, or from 40 to 50.

y1 is an integer of 0 or more, preferably an integer of 1 or more, and more preferably an integer of 5 or more. As well, y1 may be an integer of 30 or less, preferably an integer of 20 or less, more preferably an integer of 13 or less. More specifically, y1 may be an integer of from 0 to 30, preferably an integer of from 1 to 20, and still more preferably an integer of from 5 to 13.

z1 is an integer of 0 or more, and preferably an integer of 1 or more. As well, z1 may be an integer of 15 or less, and more preferably an integer of 10 or less, or 7. More specifically, z1 may be an integer of from 0 to 15, preferably an integer of from 1 to 10, and still more preferably an integer of from 1 to 7.

Preferably, y1 and z1 may satisfy a relation of y1≥z1. y1 and z1 may also satisfy a relation that y1 + z1 is an integer of from 5 to 30. Preferably, y1 + z1 may satisfy a relation that y1 + z1 is an integer of from 10 to 14.

The alcohol ethoxylate may be either a branched chain alcohol ethoxylate or a linear alcohol ethoxylate. Preferably, the alcohol ethoxylate may be a branched-chain alcohol ethoxylate.

The branched chain alcohol ethoxylate corresponds to a compound represented by the above formula (1) in which y1 is an integer of 1 or more and z1 is an integer of 1 or more. Examples of such a branched-chain alcohol ethoxylate include polyoxyethylene (40) sec-tridecyl ether. Specific examples of such a branched-chain alcohol ethoxylate include TERGITOL (registered trademark) series compounds such as Tergitol 15-S-40.

The linear alcohol ethoxylate corresponds to a compound represented by the above formula (1) in which z1 is 0. Specific examples of the linear alcohol ethoxylate include BRIJ (registered trademark) series compounds such as BrijS100. The linear alcohol ethoxylate can be represented also by the following formula (1').

H-(-CH₂-) _{y1}-O- (-CH₂-C-H₂-O-) ₓ₁-H (1')

wherein x1 is an integer of 1 or more; and
y1 is an integer of 1 or more.

The preferred examples of x1 and y1 in formula (1') are the same as the preferred examples of x1 and y1 in formula (1), respectively.

Preferably, the compound represented by formula (1) is as follows:
x1 is an integer of 40 to 100;
y1 is an integer of 0 or more;
z1 is an integer of 0 or more;
y1 ≥ z1; and
y1 + z1 is an integer of 5 to 30.

### (Preferred ranges of x1, y1 and z1 are the same as the above.)

More specifically, examples of the alcohol ethoxylate corresponding to the compound represented by formula (1) include Tergitol 15-S-40 and BrijS100. Preferably, the alcohol ethoxylate is Tergitol 15-S-40.

The "polyoxyethylene-polyoxyalkylene block copolymer" is a block copolymer that contains a polyoxyethylene block and a polyoxyalkylene block. The alkylene in the polyoxyalkylene block may be C₁₋₆ alkylene, for example. Examples of the C₁₋₆ alkylene include C₁ alkylene (methylene), C₂ alkylenes (ethylene and ethylidene), C₃ alkylenes (propylidene, propylene, trimethylene and isopropylidene), C₄ alkylenes (e.g., tetramethylene), C₅ alkylenes (e.g., pentamethylene), and C₆ alkylenes (e.g., hexamethylene). Examples of the polyoxyethylene-polyoxyalkylene block copolymer include a block copolymer having a structure of HO-[polyoxyethylene block]-[polyoxyalkylene block]-[polyoxyethylene block]-OH. The polyoxyethylene-polyoxyalkylene block copolymer may be preferably a polyoxyethylene-polyoxypropylene block copolymer.

The "polyoxyethylene-polyoxypropylene block copolymer" is a compound represented by the following formula (2):

HO- (-CH₂-CH₂-O-) ₓ₂-((-CH (-CH₃)) CH₂-O-) _{y2}-(-CH₂-CH₂-O-) _{z2}-H (2)

wherein each of x2, y2 and z2 is an integer of 1 or more.

Herein, the values of x2, y2 and z2 represent average values.

Each of x2 and z2 is an integer of 1 or more. The sum of x2 and z2 may be an integer of 2 or more, preferably an integer of 20 or more, more preferably an integer of 80 or more, and particularly preferably an integer of 150 or more. As well, the sum of x2 and z2 is an integer of 400 or less, preferably an integer of 350 or less, more preferably an integer of 300 or less, and particularly preferably an integer of 270 or less. More specifically, the sum of x2 and z2 may be an integer of 2 to 400, preferably an integer of from 20 to 350, more preferably an integer of from 80 to 300, and particularly preferably an integer of from 150 to 270.

y2 may be an integer of 1 or more, preferably an integer of 5 or more, more preferably an integer of 10 or more, still more preferably an integer of 15 or more, and particularly preferably an integer of 20 or more. As well, y2 is an integer of 200 or less, preferably an integer of 150 or less, more preferably an integer of 100 or less, still more preferably an integer of 80 or less, and particularly preferably an integer of 70 or less. More specifically, x1 may be an integer of from 1 to 200, preferably an integer of from 5 to 150, more preferably an integer of from 10 to 100, still more preferably an integer of from 15 to 80, and particularly preferably an integer of from 20 to 70.

Examples of the polyoxyethylene-polyoxypropylene block copolymer include poloxamer 188, poloxamer 388 and poloxamer 407. Specific examples of the polyoxyethylene-polyoxypropylene block copolymer include PLURONIC (registered trademark) series compounds such as Pluronic F68, Pluronic F108 and Pluronic F127.

Preferably, the compound represented by formula (2) is as follows:
x2 is an integer of 1 or more;
y2 is an integer of from 15 to 80;
z2 is an integer of 1 or more; and
x2 + z2 is an integer of from 80 to 280.

### (Preferred ranges of x2, y2 and z2 are same as the above.)

More specifically, examples of the polyoxyethylene-polyoxypropylene block copolymer corresponding to the compound represented by formula (2) include poloxamer 188 (e.g., Pluronic F68), poloxamer 108 (e.g., Pluronic F38), poloxamer 217 (e.g., Pluronic F77), poloxamer 237 (e.g., Pluronic F87), poloxamer 238 (e.g., Pluronic F88), poloxamer 288 (e.g., Pluronic F98), poloxamer 388 (e.g., Pluronic F108), and poloxamer 407 (e.g., Pluronic F127). Preferably, examples of the polyoxyethylene-polyoxypropylene block copolymer include poloxamer 188 (e.g., Pluronic F68), poloxamer 388 (e.g., Pluronic F108) and poloxamer 407 (e.g., Pluronic F127).

In certain embodiments, the nonionic surfactant may be a nonionic surfactant with an HLB of 18 or more. When the HLB value is 18 or more, the extracellular vesicles can be recovered while avoiding lysis of blood cell components more easily. In particular, it is desirable to avoid lysis of the red blood cell, a blood cell component when the extracellular vesicles are recovered, because red blood cells contain EV-related proteins such as tetraspanin proteins including CD9 and proteins that may be other impurities. The HLB value can be determined by Griffin method.

For the reference, the relationship between the nonionic surfactant (alcohol ethoxylate / polyoxyethylene-polyoxypropylene block copolymer) and the compound represented by the above formula (1) or (2) is as follows.

**Table A. Relationship between alcohol ethoxylate and compound represented by above formula (1) (first)**

| Nonionic surfactant | x1 | y1+z1 | y1 | z1 | HLB |
|---|---|---|---|---|---|
| Tergitol 15-s-40 | 40 | 10-14 | 5-13 | 1-7 | 18 |

**Table B. Relationship between alcohol ethoxylate and compound represented by above formula (1) (Second)**

| Nonionic surfactant | x1 | y1 | HLB |
|---|---|---|---|
| Brij S100 | 100 | 18 | 18 |

**Table C. Relationship between polyoxyethylene-polyoxypropylene block copolymer and compound represented by above formula (2)**

| Nonionic surfactant | x2+z2 | y2 | HLB |
|---|---|---|---|
| Pluronic F38 | 85 | 16 | >24 |
| Pluronic F68 | 153 | 29 | >24 |
| Pluronic F77 | 105 | 34 | >24 |
| Pluronic F87 | 123 | 40 | >24 |
| Pluronic F88 | 207 | 39 | >24 |
| Pluronic F98 | 236 | 45 | >24 |
| Pluronic F108 | 265 | 50 | >24 |
| Pluronic F127 | 200 | 65 | 18-23 |

In the present invention, one type of nonionic surfactant may be used, or a plurality of types (e.g., two, three, or four types) of nonionic surfactants may be used in combination.

The concentration of nonionic surfactant to act on whole blood is not particularly limited as long as the concentration of nonionic surfactant used in combination with the chelating agent can improve the recovery yield of the extracellular vesicle from whole blood. In the present invention, in light of the fact that whole blood is mixed with the nonionic surfactant and the chelating agent to give a mixture solution containing the extracellular vesicles, the nonionic surfactant and the chelating agent, the concentration of the nonionic surfactant to act on the whole blood is same as the concentration of the nonionic surfactant in such a mixture solution. Therefore, in the present invention, the concentration of the chelating agent used when the nonionic surfactant is acted on whole blood can also be defined as the concentration of the nonionic surfactant in such a mixture solution. Such a concentration is changed depending on factors such as the type and concentration of the chelating agent used in combination with the nonionic surfactant, and may be 0.01 to 10.0 weight/volume % (w/v%), for example. Preferably, the concentration of the nonionic surfactant may be 0.02 w/v% or more, 0.04 w/v% or more, 0.06 w/v% or more, 0.08 w/v% or more, 0.1 w/v% or more, 0.2 w/v% or more, or 0.5 w/v% or more. As well, such a concentration may be 8 w/v% or less, 6 w/v% or less, 5 w/v% or less, 4 w/v% or less, 3 w/v% or less, 2 w/v% or less, or 1 w/v% or less. More specifically, the concentration of the nonionic surfactant may be 0.02 to 8 w/v%, 0.04 to 6 w/v%, 0.06 to 5 w/v%, 0.08 to 4 w/v%, 0.1 to 3 w/v%, 0.2 to 2 w/v%, or 0.5 to 1 w/v%.

The chelating agent is a compound that has a coordination moiety or coordination moieties capable of forming a coordination bond or coordination bonds with a metal ion, or a salt thereof. The number of coordination moieties is preferably two or more, more preferably three or more (e.g., three or six). Examples of a coordination atom as the coordination moieties include oxygen atom, phosphorus atom, nitrogen atom, sulfur atom, and chlorine atom. The coordination atom is preferably oxygen atom or phosphorus atom, more preferably oxygen atom. Examples of a coordination group as the coordination moiety include a group having the coordination atom mentioned above. The coordination group is preferably a carboxylic acid group or a phosphate group, and more preferably a carboxylic acid group.

Examples of the chelating agent include hydroxyethyl iminodiacetic acid (HIDA), nitrilotriacetic acid (NTA), hydroxyethyl ethylenediaminetriacetic acid (HEDTA), ethylenediaminetetraacetic acid (EDTA), ethylenediaminetetra(methylene phosphonic acid) (EDTMP), and glycol ether diamine tetraacetic acid (EGTA), and salts thereof. Examples of the salt include metal salts (e.g., monovalent metal salts such as sodium salts and potassium salts, and divalent metal salts such as calcium salts and magnesium salts), inorganic salts (e.g., halide salts such as fluoride, chloride, bromide, and iodide, and ammonium salts), organic salts (e.g., ammonium salts substituted with alkyl groups) and acid addition salts (e.g., salts with inorganic acids such as sulfuric acid, hydrochloric acid, hydrobromic acid, nitric acid, and phosphoric acid, and salts of organic acids such as acetic acid, oxalic acid, lactic acid, citric acid, trifluoromethanesulfonic acid, and trifluoroacetic acid).

In certain embodiments, the chelating agent may be a chelating agent that is commonly used as a component in a blood collection vessel for clinical testing. Examples of such a chelating agent include EDTA, EGTA, NTA, HEDTA, EDTMP, HIDA, citric acid and salts thereof. In the present invention, the use of such a chelating agent is also desirable in terms of clinical application.

In the present invention, one chelating agent may be used alone, or a plurality of types of the chelating agents (e.g., two, three, or four types) may be used in combination.

The concentration of the chelating agent to act on whole blood is not particularly limited as long as the concentration can improve the recovery yield of extracellular vesicles from whole blood when the chelating agent is used in combination with the nonionic surfactant. In the present invention, in light of the fact that whole blood is mixed with the nonionic surfactant and the chelating agent to give the mixture solution containing the extracellular vesicles, the nonionic surfactant and the chelating agent, the concentration of the chelating agent to act on the whole blood is same as the concentration of the chelating agent in the mixture solution. Therefore, in the present invention, the concentration of the chelating agent to act on whole blood can also be defined as the concentration of the chelating agent in such a mixture solution. Such a concentration is changed depending on factors such as the type and concentration of the nonionic surfactant used in combination with the chelating agent, and is 10 mM to 1000 mM, for example. Preferably, the concentration of the chelating agent may be 20 mM or more, 30 mM or more, 40 mM or more, 50 mM or more, 60 mM or more, 80 mM or more, or 100 mM or more. As well, such a concentration may be 800 mM or less, 600 mM or less, 500 mM or less, 450 mM or less, 400 mM or less, 350 mM or less, or 300 mM or less. More specifically, the concentration of the chelating agent may be 20 to 800 mM, 30 to 600 mM, 40 to 500 mM, 50 to 450 mM, 60 to 400 mM, 80 to 350 mM, or 100 to 300 mM.

The mixing in a process (1) can be performed in any manner. For example, the mixing can be performed by inversion mixing and/or stirring. The order of the mixing of whole blood, the nonionic surfactants and the chelating agents, is not particularly limited. For example, the mixing may be performed by mixing whole blood with either the nonionic surfactant or the chelating agent first, and then mixing with the other. As well, the mixing may be performed by mixing whole blood with a mixture of the nonionic surfactant and the chelating agent. The mixing time is not particularly limited as long as it is sufficient time for mixing whole blood with the nonionic surfactant and the chelating agent, and may be 1 second to 30 minutes, for example, preferably 30 seconds to 5 minutes. The temperature during the mixing is 4 to 37°C, for example, and may be preferably 15 to 30°C. After the mixing, the mixture may be allowed to stand. The standing time may be 1 second to 60 minutes, for example, preferably 5 seconds to 20 minutes.

In certain embodiments, the mixing can be performed after whole blood is placed in a blood collection vessel (preferably a vacuum blood collection vessel sealed by a sealing means) containing the nonionic surfactant and the chelating agent. In this case, inversion mixing is preferred as the mixing. The blood collection vessel is described in detail below.

The separation in a process (2) can be performed in any manner. For example, the separation may be performed by any of the following (A) to (C):
(A) collection of supernatant from the mixture solution;
(B) use of a substance capable of binding to a surface marker of the extracellular vesicle in the mixture solution; or
(C) collection of supernatant from the mixture solution and use of the substance capable of binding to the surface marker of the extracellular vesicle in the supernatant.

(A) The collection of supernatant from the mixture solution is described below. In the present invention, it is demonstrated that the amount of the extracellular vesicle present in the supernatant collected from whole blood to which the chelating agent and the surfactant are added (mixture solution) is significantly improved compared to the amount of the extracellular vesicle present in supernatant collected from whole blood to which either or both of the chelating agent or the surfactant is(are) not added (EXAMPLES). Therefore, this separation enables convenient and prompt recovery of the extracellular vesicles at a high concentration (supernatant abundantly containing the extracellular vesicles) while eliminating a large amount of impurities in whole blood.

The supernatant can be collected from the mixture solution by any method. Examples of such a method include centrifugation of the mixture solution and standing of the mixture solution. Preferably, the collection of the supernatant from the mixture solution is to collect the supernatant from the mixture solution after the centrifugation of the mixture solution from the viewpoint of promptly obtaining clear supernatant. The centrifugation can be performed under a normal centrifugation condition. The condition is changed depending on factors such as a rotational radius of a centrifuge rotor, for example. As the condition, it is possible to employ a rotational speed of 100 to 100,000 x g (preferably 200 to 5,000 x g) for 10 seconds to 1 hour (preferably 1 to 10 minutes) at 40°C or less (preferably 30°C or less). The supernatant thus obtained can contain the chelating agent and the surfactant in addition to the extracellular vesicles. The concentrations of the chelating agent and the surfactant in the supernatant thus obtained can be the same as the concentrations of the chelating agent and the surfactant in the mixture solution.

(B) The use of the substance capable of binding to the surface marker of the extracellular vesicle in the mixture solution is described. In the present invention, it is demonstrated that the amount of the extracellular vesicle measured with the surface marker of the extracellular vesicle in the sample obtained from whole blood to which the chelating agent and the surfactant are added (mixture solution) is significantly improved compared to the amount of the extracellular vesicle measured with the surface marker of the extracellular vesicle in the sample collected from whole blood to which either or both of the chelating agent or the surfactant is (are) not added (EXAMPLES). Therefore, this separation enables convenient and prompt recovery of extracellular vesicles in large amounts at a high purity.

Examples of the substance capable of binding to the surface marker of the extracellular vesicle include the antibodies against the extracellular vesicle surface marker described above, aptamers, phosphatidylserine-binding proteins, and ceramide-binding proteins. In the present invention, it is possible to use single substance or a plurality of types of substances (e.g., two, three, or four types) as the substance capable of binding to the surface marker of the extracellular vesicle.

Preferably, the substance capable of binding to the surface marker of the extracellular vesicle may be an antibody against the surface marker of the extracellular vesicle, from the viewpoint of ensuring specificity to the surface marker of the extracellular vesicle, convenience of preparation and the like. Examples of the antibody include full-length antibodies (e.g., monoclonal antibodies, polyclonal antibodies) and antigen-binding fragments thereof. The antigen-binding fragment is antigen fragment that maintains capability of binding to the targeted extracellular vesicle surface marker, and may be Fab, Fab', F(ab')₂, scFv or the like. A single antibody or a plurality of types of antibodies (e.g., two, three, or four types) can be used in the present invention.

The substance (preferably antibody) capable of binding to the surface marker of the extracellular vesicle may be immobilized on a solid phase for facilitating the separation of the extracellular vesicles. Examples of the solid phase include beads and particles (e.g., sepharose beads, agarose beads, magnetic beads (magnetic particles)) and supports (e.g., plates such as plastic plates, and membranes). The substance can be immobilized on the solid phase by any method. Such a substance capable of binding to the extracellular vesicle surface marker immobilized on the solid phase is mixed with the mixture solution containing the extracellular vesicles to form a complex of the extracellular vesicle and the substance capable of binding to the extracellular vesicle surface marker. Then, the complex can be separated from the mixture solution to separate the extracellular vesicles from the mixture solution. For example, in the case of using the beads or particles as the solid phase, the extracellular vesicles can be separated from the mixture solution by centrifuging after the formation of the complex and then removing the supernatant. In the case of using magnetic beads as the solid phase, the extracellular vesicles may be separated from the mixture solution by magnetically collecting the magnetic beads after the complex formation and then removing the supernatant. In the case of using the support as the solid phase, the extracellular vesicles can be separated from the mixture solution by removing the mixture solution after the complex formation.

(C) The collection of supernatant from the mixture solution and use of the substance capable of binding to the surface marker of the extracellular vesicle in the supernatant can be carried out by the above (A) and (B) in combination.

The present invention also provides a method for analyzing the extracellular vesicle(s), including:
(1) mixing whole blood with the nonionic surfactant and the chelating agent to give the mixture solution containing the extracellular vesicle(s), the nonionic surfactant and the chelating agent;
(2) separating the extracellular vesicle(s) from the mixture solution; and
(3) analyzing the extracellular vesicle(s).

The processes (1) and (2) in the analysis method of the present invention can be performed in the same way as the processes (1) and (2) in the recovery method of the present invention.

In the process (3), the extracellular vesicle can be analyzed after the separation of the extracellular vesicle or simultaneously with the separation of the extracellular vesicle. Examples of an analyte in the analysis of the extracellular vesicle include components contained in the extracellular vesicle (e.g., components contained inside the extracellular vesicle, membrane components of the extracellular vesicle, components present on the membrane surface of the extracellular vesicle) and extracellular vesicle itself (particle).

The component contained in the extracellular vesicle can be analyzed qualitatively or quantitatively. Also, such an analysis refers to an analysis of one component or plural components. Examples of the components to be analyzed include proteins, nucleic acids (e.g., RNA and DNA), saccharides, lipids, amino acids, vitamins, polyamines and peptides. According to the present invention, the recovery yield of the extracellular vesicles is improved, enabling the components in the extracellular vesicle to be analyzed with high accuracy.

The component can be analyzed by any method known in the art.

In the case that the component to be analyzed is a protein, examples of the analysis method include immunoassay and mass spectrometry. Examples of the immunoassay include a direct competitive method, an indirect competitive method and a sandwich method. Also, examples of such an immunoassay include chemiluminescent immunoassay (CLIA) [e.g., a chemiluminescent enzyme immunoassay (CLEIA)], turbidimetric immunoassay (TIA), enzyme immunoassay (EIA) (e.g., direct competitive ELISA, indirect competitive ELISA, and sandwich ELISA), radioimmunoassay (RIA), latex agglutination reaction method, fluorescence immunoassay (FIA), and immunochromatography, Western blotting, immunostaining and fluorescence activated cell sorting (FACS). In the case that a plurality of components are analyzed, proteomic analysis may be performed.

In the case that the component to be analyzed is the nucleic acid, examples of the analysis method include hybridization methods using probes, gene amplification methods using primer (e.g., 2, 3 or 4 primers), and mass spectrometry.

In the case that the component to be analyzed is a component other than proteins or nucleic acids, examples of the analysis method include immunoassay and mass spectrometry. In the case that a plurality of components are analyzed, metabolome analysis can be performed.

The extracellular vesicle itself (particle) can also be analyzed qualitatively or quantitatively. The extracellular vesicle can be analyzed with equipment such as particle analysis equipment, electron microscope and flow cytometer, for example. In this case, it is possible to analyze the number, dimension, shape of particles of the extracellular vesicle and distribution thereof.

It has been reported that the extracellular vesicles can be involved in various diseases such as cancer (WO2014/003053; WO2014/152622; Taylor et al., Gynecologic Oncol, 100 (2008) pp13-21). Thus, the present invention is useful for diagnosis and drug discovery based on the extracellular vesicles, for example.

The present invention also provides the blood collection vessel containing the nonionic surfactant and the chelating agent.

As the blood collection vessel, any form can be used, but a tubular form is preferably used. The blood collection vessel in the tubular form has preferably a cross-section shaped into ring (e.g., circle or substantially circle). Preferably, the blood collection vessel is a blood collection vessel for clinical testing.

In addition, as the blood collection vessel, it is possible to use an unsealed blood collection vessel and a vacuum blood collection vessel sealed with a sealing means such as a rubber plug and a film sheet. The vacuum blood collection vessel sealed with the sealing means described above is preferred for easy collection of a certain volume of blood from the syringe and retention of the nonionic surfactant and the chelating agent contained in the blood collection vessel. The degree of decompression in the vacuum collection vessel can be set as appropriate according to factors such as the amount of collected blood.

Any material can be used as a material of the blood collection vessel, and a colorless and transparent material is preferably used. Examples of such a material include glasses and plastics (e.g., polyethylene terephthalate).

The size of the blood collection vessel used in the present invention can be defined by the volume of whole blood to be contained. For example, a blood collection vessel with a size suitable for holding 1 to 10 mL of whole blood is commonly used in clinical tests. Therefore, the blood collection vessel with such a size can be suitably used in the present invention. As a matter of course, the present invention can also employ a blood collection vessel with a size less than 1 mL (e.g., 0.5 mL or more and less than 1.0 mL) or more than 10 mL (e.g., more than 10 mL and 15 mL or less) as appropriate depending on the purpose.

The details of the nonionic surfactant and the chelating agent contained in the blood collection vessel are same as those described above for the method of the present invention. Forms of the nonionic surfactant and the chelating agent are not particularly limited as long as they are contained in the blood collection vessel. For example, each of the nonionic surfactant and the chelating agent may be contained in the blood collection vessel in a liquid or solid (e.g., powder) form. The nonionic surfactant and the chelating agent may also be provided in an accumulated form at the bottom in the blood collection vessel as a liquid or solid, or in a coating form or an impregnation form on the interior of the blood collection vessel (and the sealing means). Each of the nonionic surfactant and the chelating agent contained in the blood collection vessel may be one type or a plurality of types (e.g., two, three, or four types).

The nonionic surfactant and the chelating agent are preferably contained in the blood collection vessel in such amounts as to achieve the above concentrations of the nonionic surfactant and the chelating agent in the mixture solution as described above when mixed with whole blood added to the blood collection vessel. Such an amount of the nonionic surfactant is changed depending on factors such as the size of the blood collection vessel and the volume of whole blood to be added, and may be, for example, 0.1 to 2000 mg (0.1 mg to 200 mg per mL of whole blood), preferably 0.2 to 1500 mg (0.2 mg to 150 mg per mL of whole blood), more preferably 0.5 to 1000 mg (0.5 mg to 100 mg per mL of whole blood), still more preferably 1 to 700 mg (1 mg to 70 mg per mL of whole blood), and particularly preferably 2 mg to 500 mg (2 mg to 50 mg per mL of whole blood). As well, such an amount of the chelating agent is changed depending on factors such as the size of the collection vessel and the volume of whole blood to be added, and may be, for example, 3.8 to 3800 mg (3.8 to 380 mg per mL of whole blood), preferably 7.6 to 1900 mg (7.6 to 190 mg per mL of whole blood), more preferably 15.2 to 1500 mg (15.2 to 150 mg per mL of whole blood), still more preferably 30.4 to 1200 mg (30.4 to 120 mg per mL of whole blood), and particularly preferably 60.8 to 1000 mg (60.8 to 100 mg per mL of whole blood).

In certain embodiments, the chelating agent may be the above chelating agent that is commonly used as a component in the blood collection vessel for clinical testing. The use of such a chelating agent in the blood collection vessel is also desirable in terms of clinical application.

In other certain embodiments, the blood collection vessel may further contain one or more type of generic component(s) other than the chelating agent contained in the blood collection vessel for clinical testing. Thus, the blood collection vessel may further contain one or more type(s) of generic component(s) selected from the group consisting of heparin or a salt thereof, and fluorides (e.g., sodium fluoride). Examples of the salt are mentioned above.

The blood collection vessel of the present invention containing the nonionic surfactant and the chelating agent can significantly improve the recovery yield of the extracellular vesicle from whole blood, and is therefore useful for the preparation of blood samples that can be used for analysis of the extracellular vesicle. As well, the blood collection vessel of the present invention contains the chelating agent that is an anti-coagulant component contained in the blood collection vessel for ordinary blood tests as well as the nonionic surfactant that enables the reduction or avoidance of hemolysis, and is therefore useful for the preparation of versatile blood samples that can be used not only for the analysis of extracellular vesicles but also for ordinary blood tests. The blood collection vessel of the present invention can be suitably used for the method of the present invention as well, and is therefore useful for simple and prompt implementation of the method of the present invention.

### EXAMPLES

Hereinafter, the present invention will be described with reference to Examples, but the present invention is not limited to these Examples. All of percentages described in Examples are W/V%.

### (Materials and methods)

### (1) Preparation of antibody solid-phased particle (solid-phase antibody)

To 0.01 g/mL of magnetic particles in 10 mM MES buffer (pH 5.0), 0.2 mg/mL of a mouse anti-CD9 monoclonal antibody (Fujirebio Inc.) that recognizes an extracellular vesicle (EV) surface marker CD9, was added. Then, the resulting solution was gently stirred at 25°C for one hour for incubation. After the reaction, the magnetic particles were magnetically collected and washed with a washing solution (50 mM Tris buffer, 150 mM NaCl₂, 2.0% BSA, pH 7.2) in order to obtain anti-CD9 antibody solid-phased particles. Each antibody solid-phased particle was suspended in a particle diluent (50 mM Tris buffer, 1 mM EDTA·2Na, 0.1% NaN₃, 2.0% BSA, pH 7.2) in order to obtain each antibody solid-phased particle solution.

### (2) Preparation of alkaline phosphatase labeled antibody

Desalted alkaline phosphatase (ALP) was mixed with N-(4-maleimidobutyryloxy)-succinimide (GMBS) (final concentration 0.3 mg/mL) and the resulting solution was allowed to stand at 30°C for one hour for maleimidation of ALP. Next, in a coupling reactant solution (100 mM phosphate buffer, 1 mM EDTA·2Na, pH 6.3), Fab's of mouse anti-CD9 monoclonal antibody was mixed with maleimidized ALP at a molar ratio of 1: 1, and reacted at 25°C for one hour. The reactant solution was subjected to purification using Superdex 200 column chromatography (General Electric Company) to obtain ALP-labeled anti-CD9 antibody. Each ALP-labeled antibody was suspended in a labeled antibody diluent (50 mM MES buffer, 150 mM NaCl₂, 0.3 mM ZnCl₂, 1 mM MgCl₂, 0.1% NaN₃, 2.0% BSA, pH 6.8) to obtain each labeled antibody solution.

### (3) Measurement method for EV surface marker (CD9)

The measurement method for EV surface marker (CD9) in the following Examples is as follows.

20 µL of the sample for measurement and 50 µL of the anti-CD9 antibody solid-phased particle solution were dispensed into the reaction vessel and stirred. Then, the resulting solution was incubated at 37°C for 8 minutes (primary reaction), and B/F separation and washing were carried out. 50 µL of the ALP-labeled antibody was dispensed into the reaction vessel, the resulting solution was stirred and then incubated at 37°C for 8 minutes, and B/F separation and washing were carried out. Then, 200 µL of Lumipulse (registered trademark) substrate solution (Fujirebio Inc.) containing 3-(2'-spiroadamantane)-4-methoxy-4-(3"-phosphoryloxy)phenyl-1,2-dioxetane disodium salt (AMPPD) serving as a chemiluminescent substance was dispensed into the reaction chamber. The resulting solution was stirred and then incubated at 37°C for 4 minutes for the measurement of luminescence intensity using luminometer. A count value was obtained as the measured value. The luminescence intensity was measured using a fully automated chemiluminescent enzyme immunoassay system (Lumipulse L2400 (Fujirebio Inc.)).

Antibodies recognizing the same epitope in CD9 were used as the antibody solid-phased on the magnetic particle and the ALP-labeled antibody. Due to the presence of a plurality of CD9 on a single extracellular vesicle, it is possible to measure for the extracellular vesicle presenting a plurality of CD9 to be indicated as the surface marker rather than free CD9 by means of the sandwich immunoassay in which the antibodies recognizing the same epitope as the solid-phased antibodies were used as the ALP-labeled antibodies.

### Example 1: Recovery of extracellular vesicle (EV) from whole blood supplemented with chelating agent

The chelating agent was added to human whole blood. After centrifugation, CD9 in the supernatant was measured. The chelating agents used were disodium ethylenediaminetetraacetic acid (EDTA·2Na), glycol ether diamine tetraacetic acid (EGTA), nitrilotriacetic acid (NTA), hydroxyethyl ethylenediaminetriacetic acid (HEDTA), ethylenediaminetetra(methylene phosphonic acid) (EDTMP) and hydroxyethyl iminodiacetic acid (HIDA).

More specifically, 900 µL of whole blood obtained after blood collection was added to a 5 mL polypropylene test tube (manufactured by nunc) containing 100 µL of D-PBS (-) supplemented with each chelating agent. The chelating agent was added to achieve a final concentration of 15 mM after mixed with whole blood. After the addition of whole blood, the test tubes were inverted for mixing, and allowed to stand at room temperature for 30 minutes. After centrifugation (HITACHI CF5RE, 3000 rpm (2130xg), 5 minutes, room temperature), the supernatant was collected for the measurement of CD9, the surface marker protein of the extracellular vesicle (EV), by the method described in (Material and method) in order to obtain a count value. As a control (without the addition), D-PBS (-) was used for the measurement in the same way, without the addition of chelating agent.

As a result, for all of the chelating agents studied, the supernatant prepared from whole blood supplemented with the chelating agent exhibited significantly higher count values than supernatants prepared from whole blood without the chelating agent (Table 1). This indicates that the amount of EV present in the supernatant prepared from whole blood with the chelating agent is significantly increased compared to the amount of EV present in the supernatant prepared from whole blood without the chelating agent.

Therefore, the addition of the chelating agent to whole blood was revealed to significantly increase the recovery yield of EV.

**Table 1. Recovery of EV from whole blood with chelating agent**

| | Chelating agent | | | | | | |
|---|---|---|---|---|---|---|---|
| | Non-addition | Final concentration: 15 mM | | | | | |
| | | EDTA2Na | EGTA | NTA | HEDTA | EDTMP | HIDA |
| Count | 41,670 | 156,946 | 89,589 | 178,210 | 401,670 | 394,313 | 163,026 |
| vs. non-addition (%) | 100 | 376.6 | 215 | 427.7 | 963.9 | 946.3 | 391.2 |

### Example 2: Confirmation of presence and absence of hemolysis in whole blood supplemented with surfactant

A surfactant was added to human whole blood for confirmation of presence and absence of hemolysis. Tergitol 15-S-30, Brij 35, Pluronic F68, TWEEN20, and PVPK30 were used as the nonionic surfactants. Octyltrimethylammonium bromide (C8TAB) and hexadecyltrimethylammonium chloride (C16TAC) were used as cationic surfactants. N-decanoyl sarcosine sodium (N-DSS (NDS)) and N-lauroyl sarcosine sodium hydrate (NLS) were used as anionic surfactants. As amphoteric surfactants, CHAPS and N-dodecyl-N,N-dimethyl-3-ammonio-1-propanesulfonate (C12APS) were used.

More specifically, 900 µL of whole blood obtained after blood collection was added to the 5 mL polypropylene test tube (manufactured by nunc) containing 100 µL of D-PBS (-) supplemented with the surfactant. The surfactant was added to achieve a final concentration of 0.5 W/V% after mixed with whole blood. After the addition of whole blood, the test tubes were inverted for mixing, and allowed to stand at room temperature for 30 minutes. After centrifugation (3000 rpm, 5 minutes), the supernatant was collected for visual check of the presence and absence of hemolysis.

The results revealed that nonionic surfactants such as Tween, Brij, and tergitol do not cause hemolysis (Table 2).

**Table 2. Confirmation of the presence and absence of hemolysis in whole blood with surfactant**

| Surfactant | Specimen 1 | Specimen 2 |
|---|---|---|
| TWEEN20 | - | - |
| Tergitol 15-S-30 | - | - |
| Brij 35 | - | - |
| Pluronic F68 | - | - |
| C8TAB | - | - |
| C16TAC | Hemolysis | Hemolysis |
| N-DSS (NDS) | Hemolysis | Hemolysis |
| NLS | Hemolysis | Hemolysis |
| CHAPS | Hemolysis | Hemolysis |
| C12APS | Hemolysis | Hemolysis |
| PVPK30 | - | - |

### Example 3: Recovery of EV from whole blood supplemented with chelating agent and surfactant

The chelating agent and the surfactant were added to human whole blood. CD9 was measured in the supernatant after centrifugation. EDTA·2Na and EGTA were used as the chelating agent. Tergitol 15-S-40 was used as the surfactant.

First, 100 µL of D-PBS (-) with the chelating agent and 100 µL of D-PBS (-) with the surfactant were placed in the 5 mL polypropylene test tube (manufactured by nunc) to give a blood collection vessel model containing the chelating agent and the surfactant. In the preparation of this blood collection vessel model, the chelating agent was set to have a final concentration of 60 mM after mixed with whole blood. The surfactant was set to achieve a final concentration of 0.5% after mixed with whole blood.

Then, 800 µL of whole blood obtained after blood collection was added to this test tube (blood collection vessel model). After the addition of whole blood, the test tubes were inverted for mixing, and allowed to stand at room temperature for 30 minutes. After centrifugation (3000 rpm, 5 minutes), the supernatant was collected to measure CD9, the surface marker protein of extracellular vesicle (EV), by the method described in (Material and method).

For comparison, a blood collection vessel containing neither the chelating agent nor the surfactant, and a blood collection vessel containing the chelating agent without the surfactant were prepared for the measurement of CD9 in the same way.

As a result, regardless of types of the chelating agent, the supernatant prepared from whole blood with the chelating agent and the surfactant exhibited a significantly higher count value than the supernatant prepared from whole blood with the chelating agent without the surfactant (Table 3). This indicates that the amount of EV present in the supernatant prepared from whole blood with the chelating agent and the surfactant is significantly increased compared to the amount of EV present in the supernatant prepared from whole blood that is supplemented with the chelating agent without surfactant.

Therefore, the addition of the chelating agent and the surfactant to whole blood was revealed to significantly increase the EV recovery yield.

**Table 3. Recovery of EV from whole blood supplemented with chelating agent and surfactant**

| | | Chelating agent | | |
|---|---|---|---|---|
| | | Non-addition D-PBS(-) | EDTA2Na 60 mM | EGTA 60 mM |
| Count | No surfactant added D-PBS(-) | 26,148 | 51,396 | 86,959 |
| | Tergitol 15-S-40 | | 70,239 | 103,756 |
| | 0.5% | | | |
| vs. No chelating agent and surfactant added (%) | No surfactant added D-PBS(-) | 100 | 196.6 | 332.6 |
| | Tergitol 15-S-40 | | 268.6 | 396.8 |
| | 0.5% | | | |

### Example 4: Study of concentrations of chelating agent and surfactant

Various concentrations of the chelating agent and the surfactant were added to human whole blood, for the measurement of CD9 in the supernatant obtained after centrifugation.

First, 100 µL of D-PBS (-) with various concentrations of EGTA and 100 µL of D-PBS (-) with various concentrations of Tergitol 15-S-40 were placed in the 5 mL polypropylene test tubes (manufactured by nunc) to give blood collection vessel models each containing the chelating agent and the surfactant. In the preparation of this blood collection vessel model, EGTA was set to have final concentrations of 0, 10, 50, 100, and 200 mM after mixed with whole blood. Tergitol 15-S-40 was set to achieve final concentrations of 0, 0.1, 0.25, 1.0, and 2.0% after mixed with whole blood.

Then, 800 µL of whole blood obtained after blood collection was added to this test tube (blood collection vessel model). After the addition of whole blood, the test tubes were inverted for mixing, and allowed to stand at room temperature for 30 minutes. After centrifugation (3000 rpm, 5 minutes), the supernatant was collected to measure CD9, the surface marker protein of extracellular vesicle (EV), by the method described in (Material and method).

As a result, the chelating agent increased the reactivity dependently on the concentration (Table 4). The surfactant did not increase reactivity alone, but increased the reactivity in combination with the chelating agent (Table 5). The reactivity was increased with the concentration of the surfactant, in combination with the chelating agent (Tables 4 and 5). This indicates that the amount of EV present in the supernatant prepared from whole blood with the chelating agent and the surfactant increased dependently on the concentration of the chelating agent and the surfactant.

Therefore, the addition of the chelating agent and the surfactant to whole blood at high concentrations was revealed to increase the EV recovery yield.

**Table 4. Study of chelating agent concentration**

| | | | Tergitol 15-S-40 concentration (%) | | |
|---|---|---|---|---|---|
| CD9 | Chelating agent | Concentration (mM) | 0 | 0.25 | 2 |
| Count | EGTA | 0 | 3026 | 3973 | 3467 |
| | | 10 | 4671 | 5871 | 58122 |
| | | 50 | 8886 | 14303 | 71632 |
| | | 100 | 16084 | 24314 | 85885 |
| | | 200 | 21812 | 50340 | 62147 |
| vs. 0 mM | | | | | |
| EGTA (%) | EGTA | 0 | 100 | 131 | 115 |
| | | 10 | 154 | 194 | 1921 |
| | | 50 | 294 | 473 | 2367 |
| | | 100 | 532 | 804 | 2838 |
| | | 200 | 721 | 1664 | 2054 |

**Table 5. Study of surfactant concentration**

| | | | EGTA concentration (mM) | |
|---|---|---|---|---|
| CD9 | Surfactant | Concentration (%) | 0 | 50 |
| Count | Tergitol 15-S-40 | 0 | 3026 | 8886 |
| | | 0.1 | 2731 | 13056 |
| | | 0.25 | 3973 | 14303 |
| | | 1 | 3934 | 28943 |
| vs. 0% | | | | |
| Tergitol | | | | |
| (%) | Tergitol 15-S-40 | 0 | 100 | 294 |
| | | 0.1 | 90 | 431 |
| | | 0.25 | 131 | 473 |
| | | 1 | 130 | 957 |

### Example 5: Study of effects of chelating agent and surfactant

We investigated whether the chelating agent and the surfactant act on whole blood or on immune reaction.

First, 125 µL of D-PBS (-) with the chelating agent (EGTA) and 125 µL of D-PBS (-) with the surfactant (tergitol 15-S-40) were placed in the 5 mL polypropylene test tube (manufactured by nunc) to give the blood collection vessel model containing the chelating agent and the surfactant. In the preparation of this blood collection vessel model, the chelating agent and the surfactant were set to meet final concentrations listed in Table 6 after mixed with whole blood.

Then, 1000 µL of whole blood obtained after blood collection was added to this test tube (blood collection vessel model). After the addition of whole blood, the test tubes were inverted for mixing, and allowed to stand at room temperature for 30 minutes. After centrifugation (3000 rpm, 5 minutes), the supernatant was collected to measure CD9, the surface marker protein of extracellular vesicle (EV), by the method described in (Material and method).

In addition, CD9 was measured also for a solution obtained by adding 100 mM EGTA to the CD9 antibody solid-phased particle solution.

In the case of adding 100 mM EGTA to the CD9 antibody solid-phased particle solution (particle diluent), EGTA concentration during the primary reaction was 71.4 mM. In the case of adding EGTA to the blood collection vessel at a final concentration of 200 mM after mixing with whole blood, the EGTA concentration during the primary reaction was 57.1 mM. If EGTA acts during the immune reaction, the count value should be higher even when EGTA was added to the CD9 antibody solid-phased particle solution.

First, the conditions using the supernatants prepared from whole blood with EGTA at a final concentration of 200 mM [200 mM EGTA and 0% tergitol 15-s-40, 200 mM EGTA and 1% tergitol 15-S-40] achieved significantly higher count values than the conditions using supernatants prepared from whole blood without EGTA (0 mM EGTA and 0% tergitol 15-s-40) at the same concentration (see "vs. not added (%)" in Table 6). On the other hand, when EGTA was added to the CD9 antibody solid-phased particle solution, the count value was not significantly higher than that obtained when EGTA was not added to the CD9 antibody solid-phased particle solution (see "vs. no EGTA (%)" in Table 6). This indicates that the chelating agent and the surfactant act on whole blood, not the immune reaction in the CD9 assay system.

Therefore, it was demonstrated that the addition of the chelating agent and the surfactant to whole blood improves the recovery yield of extracellular vesicles.

**Table 6. Study of action by combination of chelating agent and surfactant**

| Addition to blood collection vessel | | | Addition to CD9 antibody solid-phased particle solution | |
|---|---|---|---|---|
| Specimen | Chelating agent | Surfactant | 0.5% Tergitol | 100mM EGTA + 0.5% Tergitol |
| Count average | 0mM EGTA (Not added) | 0% Tergitol (Not added) | 19398 | 13768 |
| | 200mM EGTA | 0% Tergitol (Not added) | 102897 | 116133 |
| | 200mM EGTA | 1% Tergitol | 207351 | 267054 |
| vs. non-addition (%) | 0mM EGTA | 0% Tergitol | 100 | 100 |
| | 200mM EGTA | 0% Tergitol | 530.5 | 843.5 |
| | 200mM EGTA | 1% Tergitol | 1068.9 | 1939.7 |
| vs. no EGTA (%) | 0mM EGTA | 0% Tergitol | | 71 |
| | 200mM EGTA | 0% Tergitol | | 112.9 |
| | 200mM EGTA | 1% Tergitol | | 128.8 |

| | | | | |
|---|---|---|---|---|
| All of Tergitols listed in the table refer to tergitol 15-S-40. | | | | |

### Example 6. Study of surfactant type

We investigated whether various nonionic surfactants act on whole blood. Tergitol 15-S-40, Pluronic F68, Pluronic F108, and Pluronic F127 were used as surfactants.

First, 125 µL of D-PBS (-) with EGTA and 125 µL of D-PBS (-) with each of various surfactants were placed in the 5 mL polypropylene test tube (manufactured by nunc) to give blood collection vessel models each containing the chelating agent and the surfactant. In the preparation of this blood collection vessel model, the chelating agent and the surfactant were set to meet final concentrations listed in Table 7 after mixed with whole blood.

Then, 1000 µL of whole blood obtained after blood collection was added to this test tube (blood collection vessel model). After the addition of whole blood, the test tubes were inverted for mixing, and allowed to stand at room temperature for 30 minutes. After centrifugation (3000 rpm, 5 minutes), the supernatant was collected to measure CD9, the surface marker protein of extracellular vesicle (EV), by the method described in (Material and method).

The results revealed that, regardless of the type of surfactant, the supernatant prepared from whole blood supplemented with the chelating agent and the surfactant exhibits a significantly higher count value than the supernatant prepared from whole blood supplemented with the chelating agent without surfactant (Table 7). This indicates that regardless of the type of surfactant, the amount of EV present in supernatant prepared from whole blood supplemented with the chelating agent and the surfactant is significantly increased compared to the amount of EV present in supernatant prepared from whole blood supplemented with the chelating agent without surfactant.

Therefore, the addition of chelating agents and various surfactants to whole blood was revealed to significantly increase the EV recovery yield.

**Table 7. Study of the action by combinations of chelating agent and various surfactants**

| | Surfactant | EGTA | |
|---|---|---|---|
| | | 0 mM | 200 mM |
| Count average | non-addition (D-PBS(-)) | 8414 | 30827 |
| | 1% tergitol 15-S-40 | 8898 | 117575 |
| | 1% pluronic F68 | 5749 | 559745 |
| | 1% pluronic F108 | 6412 | 654758 |
| | 1% pluronic F127 | 18681 | 236219 |
| vs. non-addition | non-addition (D-PBS(-)) | 100 | 366.4 |
| | 1% tergitol 15-S-40 | 105.8 | 1397.4 |
| | 1% pluronic F68 | 68.3 | 6652.5 |
| | 1% pluronic F108 | 76.2 | 7781.8 |
| | 1% pluronic F127 | 222 | 2807.5 |

### Industrial Applicability

For example, the present invention is useful for clinical testing of extracellular vesicles.

## Claims

1. A method for recovering an extracellular vesicle, the method comprising:
(1) mixing whole blood with a nonionic surfactant and a chelating agent to give a mixture solution containing the extracellular vesicle, the nonionic surfactant and the chelating agent; and
(2) separating the extracellular vesicle from the mixture solution.

2. The method according to claim 1, wherein the nonionic surfactant is a nonionic surfactant having a polyoxyethylene alcohol structure.

3. The method according to claim 2, wherein the nonionic surfactant having the polyoxyethylene alcohol structure is an alcohol ethoxylate.

4. The method according to claim 2, wherein the nonionic surfactant having the polyoxyethylene alcohol structure is a polyoxyethylene-polyoxyalkylene block copolymer.

5. The method according to any one of claims 1 to 4, wherein the nonionic surfactant is a nonionic surfactant with an HLB of 18 or more.

6. The method according to any one of claims 1 to 5, wherein a concentration of the nonionic surfactant in the mixture solution is 0.01 to 10 weight/volume %.

7. The method according to any one of claims 1 to 6, wherein the chelating agent is hydroxyethyl iminodiacetic acid (HIDA), nitrilotriacetic acid (NTA), hydroxyethyl ethylenediaminetriacetic acid (HEDTA), ethylenediaminetetraacetic acid (EDTA), ethylenediaminetetra (methylene phosphonic acid) (EDTMP), glycol ether diamine tetraacetic acid (EGTA), or a salt thereof.

8. The method according to any one of claims 1 to 7, wherein a concentration of the chelating agent in the mixture solution is 10 mM to 1000 mM.

9. The method according to any one of claims 1 to 8, wherein the mixing is performed after the whole blood is placed in a blood collection vessel containing the nonionic surfactant and the chelating agent.

10. The method according to any one of claims 1 to 9, wherein the separating is performed by any of the following (A) to (C):
(A) collection of a supernatant from the mixture solution;
(B) use of a substance capable of binding to a surface marker of the extracellular vesicle in the mixture solution; or
(C) collection of the supernatant from the mixture solution and use of the substance capable of binding to the surface marker of the extracellular vesicle(s) in the supernatant.

11. The method according to any one of claims 1 to 10, wherein the extracellular vesicle is exosome.

12. A method for analyzing an extracellular vesicle, the method comprising:
(1) mixing whole blood with a nonionic surfactant and a chelating agent to give a mixture solution containing the extracellular vesicle, the nonionic surfactant and the chelating agent;
(2) separating the extracellular vesicle from the mixture solution; and
(3) analyzing the extracellular vesicle.

13. A blood collection vessel comprising a nonionic surfactant and a chelating agent.

14. The blood collection vessel according to claim 13, wherein an amount of the nonionic surfactant in the blood collection vessel is 0.1 to 2000 mg.

15. The blood collection vessel according to claim 13 or 14, wherein an amount of the chelating agent in the blood collection vessel is 3.8 to 3800 mg.
